**Europäisches Patentamt**

**European Patent Office**

**Office européen des brevets**

(11) Veröffentlichungsnummer: **0 278 106 B1**

(12) **EUROPÄISCHE PATENTSCHRIFT**

(45) Veröffentlichungstag der Patentschrift:
23.08.89

(51) Int. Cl.⁴: **C07C 31/20**, C07C 29/38, C07C 29/14

(21) Anmeldenummer: **87119090.6**

(22) Anmeldetag: **23.12.87**

(54) **Verfahren zur Herstellung von 2,2-Dimethylpropandiol-(1,3).**

(30) Priorität: **30.12.86 DE 3644675**

(43) Veröffentlichungstag der Anmeldung:
**17.08.88 Patentblatt 88/33**

(45) Bekanntmachung des Hinweises auf die Patenterteilung:
**23.08.89 Patentblatt 89/34**

(84) Benannte Vertragsstaaten:
**DE FR GB IT SE**

(56) Entgegenhaltungen:
**DE-A- 1 518 784**
**DE-A- 1 957 591**
**DE-C- 1 057 083**
**US-A- 4 393 251**

**CHEMICAL ABSTRACTS, Band 73, Nr. 7, 17.
August 1970, Seite 282, Spalte 2,
Zusammenfassungsnr. 34798p, Columbus, Ohio, US; &
JP - A - 70 10926**

(73) Patentinhaber: **HOECHST AKTIENGESELLSCHAFT,
Postfach 80 03 20, D-6230 Frankfurt am Main 80(DE)**

(72) Erfinder: **Breitkopf, Norbert, Heinrichstrasse 10,
D-4200 Oberhausen 12(DE)**
Erfinder: **Höfs, Wolfgang, Dipl.-Ing., Höhenweg 18,
D-4200 Oberhausen 11(DE)**
Erfinder: **Kalbfell, Heinz, Dipl.-Ing., Eichenstrasse 20,
D-4235 Schermbeck(DE)**
Erfinder: **Thönnessen, Franz, Dr. Dipl.-Chem.,
Lützowstrasse 49, D-4200 Oberhausen 11(DE)**
Erfinder: **Lappe, Peter, Dr. Dipl.-Chem., Eickenhof 34,
D-4220 Dinslaken(DE)**
Erfinder: **Springer, Helmut, Dipl.-Ing.,
Drostenkampstrasse 34, D-4200 Oberhausen 11(DE)**

ACTORUM AG

## Beschreibung

Die vorliegende Erfindung betrifft ein Verfahren zur Herstellung von 2,2-Dimethylpropandiol-(1,3) aus Isobutyraldehyd und Formaldehyd unter Verwendung von Tri-n-propylamin als Katalysator, Hydrierung des Reaktionsgemisches und anschließende Destillation des Hydrierungsproduktes in Gegenwart von Isobutanol und Wasser.

Es ist bekannt, aus Isobutyraldehyd und Formaldehyd durch Aldoladdition 2,2-Dimethyl-3-hydroxypropanal herzustellen und den Oxyaldehyd anschließend zum entsprechenden Diol zu hydrieren. Als Katalysatoren für die Aldoladdition finden basisch reagierende Verbindungen Verwendung. Weit verbreitet ist der Einsatz von Alkalihydroxiden, Erdalkalihydroxiden und Alkalicarbonaten. Daneben gelangen auch Amine, insbesondere tertiäre Mono- oder Polyamine, z.B. Diamine, zur Anwendung. Ein derartiges Verfahren ist z.B. in der DE 19 57 591 B2 beschrieben. Zur Herstellung von 2,2-Dimethylpropandiol-(1,3) setzt man Isobutyraldehyd und Formaldehyd in Gegenwart von tertiären Aminen um und hydriert das anfallende Reaktionsgemisch mit Wasserstoff. Als geeignet werden u.a. folgende Amine genannt: Trimethyl-, Triethyl-, Methyl-diethyl-, Methyl-diisopropylamin, Tributylamin. In den Beispielen wird als Katalysator ausschließlich Triethylamin verwendet.

Der Einsatz von Aminen als Additionskatalysatoren hat den Vorteil, daß Nebenprodukte, die bei Verwendung anderer basischer Katalysatoren gebildet werden, nicht entstehen. Dem steht jedoch entgegen, daß tertiäre Amine teure Reagenzien sind, die Wirtschaftlichkeit des Prozeßes daher eine möglichst verlustfreie Wiedergewinnung des Amins aus dem Reaktionsgemisch verlangt. Eine quantitative Abtrennung der Amine ist auch deshalb erforderlich, weil mit Aminen auch nur in geringen Mengen verunreinigtes Diol für viele Verwendungszwecke ungeeignet ist. Die Herstellung von reinem 2,2-Dimethylpropandiol-(1,3) verlangt daher einen sehr hohen Trennaufwand.

Es bestand somit die Aufgabe, einen Prozeß zu entwickeln, der technisch einfach durchzuführen ist und mit wirtschaftlich vertretbaren Mitteln die Gewinnung von 2,2-Dimethylpropandiol-(1,3) durch Amin katalysierte Aldoladdition ermöglicht.

Die Erfindung besteht in einem Verfahren zur Herstellung von 2,2-Dimethylpropandiol-(1,3) durch Addition von Isobutyraldehyd und Formaldehyd in Gegenwart von tertiären Aminen als Katalysator, Hydrierung des Reaktionsgemisches und anschließende Destillation des Hydrierproduktes. Es ist dadurch gekennzeichnet, daß als Katalysator Tri-n-propylamin eingesetzt wird und die Destillation in Gegenwart von Isobutanol erfolgt.

Das neue Verfahren liefert sehr reines 2,2-Dimethylpropandiol-(1,3), das Amin selbst in Spuren nicht mehr enthält. Ein weiterer Vorzug der erfindungsgemäßen Arbeitsweise ist der geringe Aminverlust über die Gesamtheit der einzelnen Reaktionsschritte. Von dem ursprünglich eingesetzten Amin werden bis zu 97 Gew.-% zurückgewonnen.

Die unerwarteten Vorteile des Verfahrens beruhen auf der ausgezeichneten Eignung von Tri-n-propylamin als Additionskatalysator und von Isobutanol als Lösungsmittel und Teil des Reaktionsmediums in jedem einzelnen Verfahrensschritt, sowie auf der Bildung verschiedener Azeotrope, die zwei oder alle drei der Komponenten Tri-n-propylamin, Isobutanol und Wasser enthalten. Ihre Ausbildung stellt sicher, daß eine vollständige Abtrennung insbesondere des Tri-n-propylamins als dem höchstsiedenden Bestandteil aber auch der übrigen Komponenten erreicht wird und die thermische Belastung des Reaktionsproduktes gering ist. Als weiterer Vorteil des neuen Verfahrens ist hervorzuheben, daß das Amin zurückgeführt und als Katalysator wieder eingesetzt werden kann.

Tri-n-propylamin ist im Verlauf des mehrstufigen Verfahrens von Anfang an im Reaktionsgemisch zugegen. Das gleiche gilt für das Wasser, denn Formaldehyd wird üblicherweise als wäßrige Lösung eingesetzt. Auch Isobutanol kann man dem ursprünglichen Reaktionsgemisch zusetzen. Es ist aber auch möglich, die Additionsreaktion mit überschüssigem Isobutyraldehyd oder in Gegenwart eines Isobutyraldehyd/Isobutanol-Gemisches durchzuführen. Bei der sich an die Additionsreaktion anschließenden Hydrierung wird dann aus dem Aldehyd Isobutanol gebildet. Schließlich kann Isobutanol auch dem Hydrierprodukt vor dessen Destillation zugesetzt werden. Die Isobutyraldehyd- bzw. Isobutanolmenge ist so zu bemessen, daß in der Destillationsstufe das Verhältnis von Isobutanol zu übrigem Einsatzmaterial (in Gewichtsteilen) 1 : 40 bis 1 : 200, vorzugsweise 1 : 80 bis 1 : 100 beträgt.

In der ersten Reaktionsstufe läßt man Formaldehyd und Isobutyraldehyd miteinander reagieren. Die Aldehyde können im molaren Verhältnis umgesetzt werden, jedoch ist es auch möglich, einen der beiden Reaktionspartner im Überschuß anzuwenden. Zweckmäßig setzt man Formaldehyd als wäßrige Lösung (Aldehydgehalt: 37 Gew.-%) ein. Die Reaktion erfolgt bei Temperaturen zwischen 20 und 100°C, zweckmäßig arbeitet man bei 80 bis 95°C. Im allgemeinen wird die Reaktion bei Normaldruck durchgeführt, jedoch kann man auch erhöhten Druck anwenden. Neben Wasser aus der Formaldehydlösung und dem gegebenenfalls zugesetzten Isobutanol sind weitere Lösungsmittel nicht erforderlich.

Als Katalysator wird erfindungsgemäß Tri-n-propylamin verwendet. Es ist im Reaktionsgemisch in einer Menge von 1 bis 20, vorzugsweise 2 bis 12 Mol.-%, bezogen auf Isobutyraldehyd, enthalten.

Die praktische Durchführung der Additionsreaktion erfolgt in einem Rührkessel oder in einem Reaktionsrohr, das zur besseren Durchmischung der Reaktanten mit Füllkörpern beschickt ist. Die Umsetzung verläuft exotherm, sie kann durch Erhitzen beschleunigt werden. Das anfallende Reaktionsgemisch wird ohne vorherige Trennung in seine Bestandteile oder Abtrennung einzelner Komponenten katalytisch hydriert. Die Anlagerung von Wasserstoff kann in der Gasphase oder auch in flüssiger Phase erfolgen. Als Katalysatoren eignen sich insbesondere Nickel-Trägerkatalysatoren, die gegebenenfalls noch weitere aktive Metalle wie Kupfer oder Chrom und darüber hinaus Aktivatoren enthalten können.

Das Hydrierprodukt führt man einer kontinuierlich arbeitenden Fraktionierkolonne mit 40 bis 120, vorzugsweise 50 bis 70 Böden zu. Die Kolonne wird mit zwei Seitenabzügen betrieben, wobei Temperatur, eingesetzte Menge Hydrierprodukt und abgezogene Mengen Destillat so aufeinander abgestimmt werden, daß im Kolonnensumpf 2,2-Dimethylpropandiol-(1,3) in einer Reinheit von >99% zurückbleibt. An der unteren Seitenabnahme werden Tri-n-propylamin und Restmengen Isobutanol abgenommen, an der oberen Seitenabnahme wird ein Zweiphasensystem abgezogen, dessen organische Phase hauptsächlich aus Isobutanol besteht. Am Kopf der Kolonne fällt Methanol an, das zusammen mit leicht siedenden Nebenprodukten ausgeschleust wird. Reaktionswasser und das restliche Methanol werden über die Wasserphase des oberen Seitenabzugs abgetrennt.

Die neue Arbeitsweise zeichnet sich durch verfahrenstechnische Einfachheit und hohe Wirtschaftlichkeit aus. Diese Vorteile gehen darauf zurück, daß der Einsatzstoff in zwei Destillationsstufen aufgearbeitet wird. Grundlage hierfür ist, daß durch Rückführung der am oberen Seitenabzug anfallenden Isobutanol-Phase auf den Einsatzboden der Kolonne das System durch Ausbildung des Azeotrops Isobutanol/$H_2O$ (90°C mit 33 % $H_2O$) an Wasser verarmt. Daher kann Tri-n-propylamin am unteren Seitenabzug ausgeschleust werden, obwohl Tri-n-propylamin mit Wasser ein binäres Azeotrop (97°C mit 44,3 % $H_2O$) und auch ein ternäres Gemisch (91°C mit 50 % Isobutanol, 19 % Tri-n-propylamin, 31 % $H_2O$) bildet. Je nach Wasser-Gehalt des Einsatzmaterials und dessen Zusammensetzung muß die Menge des zurückgeführten Butanols so bemessen sein, daß die gesamte Wassermenge am oberen Seitenabzug anfällt.

Das neue Verfahren wird durch die folgenden Beispiele näher erläutert.

Beispiel 1

Durch kontinuierliche Destillation von rohem 2,2-Dimethylpropandiol-1,3, das 56 Gew.-% 2,2-Dimethylpropandiol-(1,3) und daneben 20 Gew.-% Isobutanol, 13 Gew.-% Wasser, 7 Gew.-% Tri-n-propylamin, 3 Gew.-% Methanol, und 1 Gew.-% andere Komponenten enthält, an einer Füllkörperkolonne mit 60 theoretischen Böden (Abtriebsteil 10 Böden, Verstärkerteil 50 Böden, Seitenabnahmen jeweils am 20. und am 40. Boden) gewinnt man als Sumpfprodukt 2,2-Dimethylpropandiol-(1,3) in einer Reinheit von mehr als 99 %. Der größere Anteil des eingesetzten Methanols wird am Kopf der Kolonne in Form eines Produktes, das etwa 85 Gew.-% Methanol und daneben Vorlaufkomponenten, Isobutanol und Wasser enthält, abgezogen. Bei einem Verhältnis von eingesetztem Gemisch zu rück geführtem Butanol von 100 : 70 erhält man an der oberen Seitenabnahme Isobutanol und Wasser und an der unteren Seitenabnahme 97% des eingesetzten Tri-n-propylamin in etwa 90 %iger Reinheit. Das Amin kann ohne weitere Reinigung in die Aldolisierungsstufe zurückgeführt werden.

Beispiel 2

Es wird unter den Bedingungen des Beispiels 1 gearbeitet, jedoch bei einem Verhältnis von Einsatzgemisch zu rückgeführtem Butanol von 100 : 30. In diesem Fall wird nur eine unvollständige Trennung der Komponenten Tri-n-propylamin, Isobutanol und Wasser erreicht.

Beispiel 3 (Vergleichsbeispiel)

Rohes 2,2-Dimethylpropandiol-(1,3), das 47,6 Gew.-% 2,2-Dimethylpropandiol-(1,3), 33,3 Gew.-% Isobutanol, 12,4 Gew.-% Wasser, 4,8 Gew.-% Triethylamin und 1,9 Gew.-% Methanol enthält, wird an einer Füllkörperkolonne destilliert, die 70 theoretischen Böden aufweist und eine Seitenabnahme hat. Selbst bei einem Rücklaufverhältnis von > 100 : 1 und einem Destillatabzug am Kopf der Kolonne von etwa 1 % kann eine ausreichende Trennung von Methanol und Triethylamin nicht erreicht werden. Überdies erweist sich bei dieser Verfahrensführung die gute Löslichkeit des Triethylamins im Wasser als nachteilig, denn die gesamte Seitenabzugsmenge, d.h. die organische und die wäßrige Phase müssen zur Synthese rezirkuliert werden.

## Patentansprüche

1.) Verfahren zur Herstellung von 2,2-Dimethylpropandiol-(1,3) durch Addition von Isobutyraldehyd und Formaldehyd in Gegenwart von tertiären Aminen als Katalysator, Hydrierung des Reaktionsgemisches und anschließende Destillation des Hydrierproduktes, dadurch gekennzeichnet, daß als Katalysator Tri-n-propylamin eingesetzt wird und die Destillation in Gegenwart von Isobutanol erfolgt.

2.) Verfahren nach Anspruch 1, dadurch gekennzeichnet, daß Tri-n-propylamin in einer Menge von 1 bis 20, vorzugsweise 2 bis 12 Mol-%, bezogen auf Isobutyraldehyd, eingesetzt wird.

3.) Verfahren nach Anspruch 1, dadurch gekennzeichnet, daß in der Destillationsstufe das Verhältnis von Isobutanol zu übrigem Einsatzmaterial (in Gewichtsteilen) 1 : 40 bis 1 : 200, vorzugsweise 1 : 80 bis 1 : 100 beträgt.

## Claims

1. A process for the preparation of 2.2-dimethylpropanediol-(1.3) by the addition of isobutyraldehyde and formaldehyde in the presence of tertiary amines as catalysts, hydrogenation of the reaction mixture and subsequent distillation of the hydrogenation product, characterised in that tri-n-propylamine is used as a catalyst and distillation takes place in the presence of isobutanol.

2. A process according to claim 1, characterised in that tri-n-propylamine is used in an amount of 1 to 20, preferably 2 to 12 mol%, related to the isobutyraldehyde.

3. A process according to claim 1, characterised in that in the distillation stage the ratio of isobutanol to the other feed material (in parts by weight) is 1:40 to 1:200, preferably 1:80 to 1:100.

**Revendications**

1. Procédé pour la fabrication de 2,2-diméthyl-propanediol-1,3 par addition d'isobutyraldéhyde et de formaldéhyde en présence d'amines tertiaires comme catalyseurs, hydrogénation du mélange de réaction suivie de distillation du produit d'hydrogénation, caractérisé en ce que l'on utilise la tri-n-propylamine comme catalyseur et on effectue la distillation en présence d'isobutanol.

2. Procédé selon la revendication 1, caractérisé en ce que la tri-n-propylamine est mise en jeu en quantité de 1 à 20 mol%, de préférence 2 à 12 mol%, par rapport à l'isobutyraldéhyde.

3. Procédé selon la revendication 1, caractérisé en ce que le rapport de l'isobutanol à l'autre matière mise en jeu (en parties en poids) dans l'étape de distillation est de 1:40 à 1:200, de préférence de 1:80 à 1:100.